(19) Europäisches Patentamt
European Patent Office
Office européen des brevets

(11) Veröffentlichungsnummer : **0 058 765**
**B1**

(12) # EUROPÄISCHE PATENTSCHRIFT

(45) Veröffentlichungstag der Patentschrift : **20.02.85**

(51) Int. Cl.[4] : **A 61 K 9/32**

(21) Anmeldenummer : **81109502.5**

(22) Anmeldetag : **04.11.81**

(54) **In Magensaft lösliche oder quellbare Überzugsmasse und ihre Verwendung in einem Verfahren zum Überziehen von Arzneiformen.**

(30) Priorität : **20.02.81 DE 3106449**

(43) Veröffentlichungstag der Anmeldung : **01.09.82 Patentblatt 82/35**

(45) Bekanntmachung des Hinweises auf die Patenterteilung : **20.02.85 Patentblatt 85/08**

(84) Benannte Vertragsstaaten : **AT BE CH DE FR GB IT LI NL SE**

(56) Entgegenhaltungen :
**EP-A- 0 027 850**
**FR-A- 2 145 642**
**GB-A- 475 132**
**PRODUCT LICENSING INDEX, Nr.85, Suppl., Mai 1971, Havant (GB) K.R. HOLLISTER et al.: "Amphilic copolymers and silver halide peptizers", Seiten 30-32**
**CHEMICAL ABSTRACTS, Band 93, Nr.4, Juli 1980, Seite 18, Zusammenfassung Nr.27071f, Columbus, Ohio (US)**

(73) Patentinhaber : **Röhm GmbH**
**Kirschenallee Postfach 4242**
**D-6100 Darmstadt 1 (DE)**

(72) Erfinder : **Markert, Gerhard, Dr.**
**Leipziger Strasse 25**
**D-6105 Ober-Ramstadt (DE)**
Erfinder : **Dreher, Dieter**
**Waldstrasse 2 A**
**D-6101 Bickenbach (DE)**
Erfinder : **Lehmann, Klaus, Dr.**
**Schillerstrasse 85**
**D-6101 Rossdorf 1 (DE)**
Erfinder : **Siol, Werner, Dr.**
**Mühlbergstrasse 4**
**D-6102 Pfungstadt (DE)**
Erfinder : **Rauch, Hubert**
**Odenwaldstrasse 6**
**D-6108 Weiterstadt 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 058 765 B1

**Beschreibung**

Die Erfindung liegt auf dem Gebiet der magensaftlöslichen Überzugsmassen für Arzneiformen, enthaltend als Bindemittel ein Emulsionspolymerisat aus Aminoalkylestern der Acryl- oder Methacrylsäure und gegebenenfalls weiteren Comonomeren, sowie weitere übliche flüssige oder feste Zusätze für Arzneimittelüberzüge. Bindemittel dieser Art sind aus der DE-PS 2 135 073 bekannt. Die zur Herstellung der Emulsionspolymerisate verwendeten Aminoalkylester enthalten durchweg zwei Kohlenstoffatome zwischen dem Amino-Stickstoffatom und dem Ester-Sauerstoffatom. Als einziges von dieser Struktur abweichendes Comonomeres wird der 4-(Dimethylamino)-butylester genannt. Bei der Herstellung von Emulsionspolymerisaten mit diesen Monomeren können Schwierigkeiten auftreten, die sich aus der leichten Wasserlöslichkeit dieser Monomeren, insbesondere des am häufigsten verwendeten Dimethylaminoäthylmethacrylats ergeben. Ein Teil der Monomeren kann für sich allein in der Wasserphase zu wasserlöslichen Homopolymerisaten polymerisieren, die selbst dann, wenn sie im Laufe der Polymerisation an Latexpartikel gebunden oder in diese eingeschlossen werden, das Löslichkeitsverhalten der damit hergestellten Arzneimittelüberzüge erheblich beeinflussen. Weiterhin neigen diese Monomeren mit kurzen oder unverzweigten Alkylengruppen im Aminoalkylrest zur Hydrolyse, die so lange eintreten kann, bis das Monomere in das Emulsionspolymerisat eingebaut ist. Bei der Hydrolyse entsteht Acryl- oder Methacrylsäure, die ebenfalls einpolymerisiert wird und die Löslichkeitseigenschaften verändert. Wegen dieser Nachteile konnten Emulsionspolymerisate, die zu mehr als 55 Gew.-% aus derartigen Aminoalkylestern aufgebaut sind, gar nicht und solche, die mehr als 30 Gew.-% der Aminoalkylester enthalten, nur mit Schwierigkeiten hergestellt werden.

Nach Chemical Abstracts, Band 93, Referat 27071 sind Polymerisate und Mischpolymerisate des p-Dialkylaminobenzyl(meth)acrylats durch Emulsionspolymerisation herstellbar. Der Einsatz derartiger Emulsionspolymerisate in Überzugsmassen für Arzneiformen ist aus dieser Veröffentlichung und der zugrundeliegenden Czechoslowakischen Patentschrift 180 820 nicht bekannt. Sie gab auch keine Hinweise, wie die Nachteile und Schwierigkeiten, die sich bei der Herstellung von Arzneimittelüberzügen gemäß DE-PS 21 35 073 gezeigt haben, überwunden werden könnten.

Es wurde nun gefunden, daß Aminoester der nachfolgend formelmäßig dargestellten Struktur weniger wasserlöslich als die oben genannten Aminoester sind, in monomerer Form weniger als diese zur Hydrolyse neigen und wesentlich leichter in Emulsionspolymerisate übergeführt werden können. Dies zeigt sich vor allem darin, daß man Emulsionspolymerisate mit mehr als 55 Gew.-% Einheiten dieser Ester und sogar ihre reinen Homopolymerisate durch Emulsionspolymerisation herstellen kann.

Gegenstand der Erfindung sind Überzugsmassen für Arzneiformen, die als Bindemittel die genannten Emulsionspolymerisate sowie weitere übliche flüssige oder feste Zusätze für Arzneimittelüberzüge enthalten.

Die am Aufbau der neuen Emulsionspolymerisate beteiligten Aminoester haben die Struktur

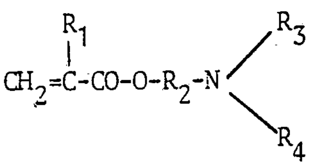

wobei $R_1$ ein Wasserstoffatom oder eine niedere Alkylgruppe, $R_2$ eine Alkylengruppe mit wenigstens 3 in gerader Kette zwischen dem Amino-Stickstoffatom und dem Ester-Sauerstoffatom angeordneten Kohlenstoffatomen, von denen wenigstens eines tertiär oder quartär ist, und $R_3$ und $R_4$ niedere Alkylreste bedeuten oder zusammen mit dem Amino-Stickstoffatom einen heteroaliphatischen Ring bilden. Als tertiäre oder quartäre Kohlenstoffatome innerhalb der geraden Kette von wenigstens 3 Kohlenstoffatomen zwischen dem Amino-Stickstoffatom und dem Ester-Sauerstoffatom werden solche Kohlenstoffatome angesehen, die mit einem bzw. zwei weiteren Kohlenstoffatomen verbunden sind. Bevorzugte Beispiele für Monomere dieser Struktur sind 3-Dialkylamino-2,2-dimethylpropylacrylat und -methacrylat. Die Aminoalkylester sind am Aufbau des Emulsionspolymerisats in einer Menge von wenigstens 5 Gew.-% beteiligt, wobei man im allgemeinen noch keine in Magensaft lösliche, sondern nur darin quellbare Überzugsmittel erhält, sofern nich erhebliche Mengen hydrophiler Comonomerer am Aufbau beteiligt sind. Die Homopolymerisate der Aminoalkylester sind zum Teil schon oberhalb des pH-Wertes des Magensaftes löslich. Der bevorzugte Mengenanteil der Aminoester am Aufbau der Emulsionspolymerisate liegt zwischen 10 und 90 Gew.-%.

Als Comonomere neben den Aminoestern kommen vor allem wasserunlösliche Monomere in Betracht und unter diesen vorzugsweise die Ester der Acryl- und Methacrylsäure. Die Acrylsäureester enthalten vorzugsweise Esteralkylreste mit 1 bis 8 C-Atomen ; die Methacrylsäureester können Alkylreste mit 1 bis 18 C-Atomen enthalten. Neben oder anstelle dieser bevorzugten Monomeren können andere wasserunlösliche ungesättigte polymerisierbare Verbindungen, wie Styrol oder Vinylester von organischen Carbonsäuren sowie Malein-, Fumar- oder Itakonsäureester in den Emulsionspolymerisaten

enthalten sein. Wasserlösliche Comonomere, wie Vinylpyrrolidon, Hydroxyester von ungesättigten polymerisierbaren Carbonsäuren oder Acryl- oder Methacrylamid oder deren N-alkylsubstituierte Derivate bilden, sofern sie am Aufbau des Emulsionspolymerisats beteiligt sind, im allgemeinen Anteile unter 20 Gew.-%. Ungesättigte Carbonsäuren wie Acryl-, Methacryl-, Malein- oder Itakonsäure oder deren wasserlösliche Salze liegen in der Regel in einem Anteil von höchstens 3 % vor. Art und Menge der Monomerbestandteile sind grundsätzlich so gewählt, daß das Emulsionspolymerisat im pH-Bereich des Magensaftes, d. h. zwischen pH 2 und 4, löslich oder zumindest quellbar ist. Die Quellbarkeit muß ausreichen, um einen Arzneimittelüberzug für Wirkstoffe wenigstens diffusionsdurchlässig zu machen.

Bekanntlich hängen die Härte und die Elastizität des aus einem Emulsionspolymerisat hergestellten Films von der Polymerisatzusammensetzung ab und können den jeweiligen anwendungstechnischen Bedürfnissen entsprechend durch geeignete Kombinationen von hartmachenden Monomeren, wie Methylmethacrylat, und weichmachenden Monomeren, zu denen z. B. Butylacrylat sowie die Mehrzahl der erfindungsgemäß eingesetzten Aminoalkylester gehören, in bekannter Weise eingestellt werden.

Das Molekulargewicht des Emulsionspolymerisats liegt im allgemeinen über 10 000 und sollte für den Fall, daß das Emulsionspolymerisat in Pulverform oder als organische Lösung verarbeitet wird, nicht über 1 Million liegen. Das Emulsionspolymerisat hat in Latexform die übliche Teilchengröße im Bereich von 0,03 bis 3 μm und kann in Pulverform Aggregate von wesentlich größerem Korndurchmesser bilden. Es ist jedoch von Vorteil, wenn derartige Aggregate nicht verglast sind, sondern aus lose aggregierten Feinpartikeln bestehen und beim Einrühren in Wasser oder andere flüssige Medien wieder in wesentlich feinere Partikel zerfallen. Dadurch ist das Pulver in organischen Lösungsmitteln rasch löslich.

Die Emulsionspolymerisate sind nach den an sich bekannten Methoden der wäßrigen Emulsionspolymerisation herstellbar und enthalten die für diese Verfahren typischen Hilfsmittel in den gebräuchlichen Mengen. Sie enthalten bevorzugt anionische oder nichtionische Emulgiermittel oder Gemische dieser Emulgiermittelarten, jedoch können Emulsionspolymerisate, die nach bekannten Verfahren emulgatorfrei hergestellt worden sind, ebenfalls eingesetzt werden. Der Gehalt an Emulgiermitteln kann, bezogen auf die wäßrige Phase, im Bereich von 0,1 bis 10 Gew.-% liegen. Der Gehalt an anionischen Emulgiermitteln liegt vorzugsweise zwischen 0,01 und 2 Gew.-% und der Gehalt an nichtionischen Emulgiermitteln zwischen 0,1 und 5 Gew.-% ; die letzteren können auch nach der Herstellung des Emulsionspolymerisats zugesetzt worden sein. Der Feststoffgehalt der Dispersion liegt vorzugsweise im Bereich von 20 bis 50 Gew.-%. Besonders bevorzugt sind Emulsionspolymerisate, die nach dem Monomerzulauf- oder Emulsionszulauf-Verfahren hergestellt worden sind. Der pH-Wert der wäßrigen Polymerisatdispersion liegt im allgemeinen zwischen 7 und 12 und vorzugsweise zwischen 8 und 10.

Die wäßrige Dispersion des Emulsionspolymerisats kann als solche als Bindemittel in eine wäßrige Überzugsmasse eingearbeitet werden. Man kann jedoch auch nach an sich bekannten Methoden, beispielsweise durch Ausfällung, Sprühtrocknung oder Gefriertrocknung, das Emulsionspolymerisat in Pulverform gewinnen und unmittelbar oder in Form einer organischen Lösung als Bindemittel für Überzugsmassen verwenden.

Obwohl es Fälle gibt, in denen die wäßrige Polymerdispersion als solche zur Herstellung eines Arzneimittelüberzugs eingesetzt wird, enthält die Mehrzahl der praktisch verwendeten Überzugsmassen für Arzneiformen eine mehr oder weniger große Zahl von flüssigen oder festen Zusätzen, die auch erfindungsgemäß verwendet werden. Unter solchen Zusätzen sind insbesondere Pigmente, Füllstoffe, lösliche Farbstoffe, Weichmachungsmittel für das Bindemittel, Glanz- und Poliermittel, Geschmackszusätze, Lichtschutzzusätze, Entschäumungsmittel und Verlaufmittel zu verstehen. Diese Zusätze können in die wäßrige Dispersion eingearbeitet oder dem pulverförmigen Emulsionspolymerisat oder einer daraus bereiteten Lösung in einem organischen Lösungsmittel zugesetzt werden.

Für die Beschichtung von Arzneiformen mit den Emulsionspolymerisaten oder daraus bereiteten Überzugsmassen können gemäß der Erfindung verschiedene Verfahren angewendet werden. So kann die wäßrige Dispersion des Emulsionspolymerisats oder eine wäßrige Überzugsmasse, die aus der Dispersion durch geeignete Zusätze hergestellt worden ist, durch das Kesseldragierverfahren oder in einer Wirbelschichtapparatur in an sich bekannter Weise auf Arzneiformen aufgebracht werden.

Man verfährt dabei wie in der DE-PS 2 135 073 beschrieben. Geringe Mengen flüchtiger organischer Flüssigkeiten, wie z. B. niedere aliphatische Alkohole, Ketone, Ester, die vorübergehend als Weichmachungsmittel wirken und die Filmbildung erleichtern, können mitverwendet werden.

Andere Beschichtungsverfahren gehen von dem aus der Wasserphase isolierten Emulsionspolymerisat aus. Es kann in feinteiliger Pulverform allein oder nach Abmischung mit üblichen festen Zusatzstoffen zum Überziehen von Arzneiformen verwendet werden. Als Arzneiformen sind z. B. auch Wirkstoffkristalle anzusehen, die mit dem pulverförmigen Emulsionspolymerisat oder mit einer durch Abmischung mit festen Zusatzstoffen hergestellten pulverförmigen Überzugsmasse zu Gerüsttabletten verpreßt werden. Auch Granulierungsverfahren mit solchen wirkstoffhaltigen Pulvergemischen sind durchführbar. Man kann jedoch auch das pulverförmige Überzugsmittel im Wirbelschichtverfahren bei erhöhter Temperatur auf Arzneiformen aufbringen, wobei die Partikel zu einer geschlossenen Schicht zusammensintern bzw. zusammenschmelzen.

Ein vorteilhaftes Beschichtungsverfahren ist die Thermogelierung. Beim Thermogelierungsverfahren wird das pulverförmige Überzugsmittel in einer wäßrigen Lösung eines Weichmachungsmittels suspendiert, wobei das Weichmachungsmittel so gewählt ist, daß sich das Emulsionspolymerisat darin

bei Raumtemperatur nicht löst, aber beim Erwärmen nach dem Verdampfen des Wassers eine gelartig feste Masse bildet. Die Suspension kann im Dragierkessel oder in der Wirbelschichtapparatur auf Arzneiformen aufgebracht und in der Wärme auf ihrer Oberfläche zu einer festen Überzugsschicht geliert werden. Bei einer anderen Ausführungsform dieses Verfahrens werden die Arzneiformen mit der wäßrigen Lösung des Weichmachungsmittels befeuchtet und das pulverförmige Überzugsmittel eingestreut. Auch in diesem Fall tritt auf der Arzneiformoberfläche die Thermogelierung zu einer geschlossenen Schicht ein. Geeignete Weichmachungsmittel sind z. B. schwerflüchtige Polyhydroxyverbindungen, wie Polyäthylenglykole, Zuckeralkohole, oder Zitronensäureester. Die Menge des Weichmachungsmittels wird so gewählt, daß es für den Vorgang der Lösung bzw. Gelierung des Emulsionspolymerisats gerade ausreicht und daß nach dem Abkühlen die Überzugsschicht fest und nicht klebrig ist.

Eine weitere bevorzugte Anwendungsform der Überzugsmassen gemäß der Erfindung besteht in der Herstellung einer organischen Lösung aus dem pulverförmigen Emulsionspolymerisat, der in an sich bekannter Weise weitere übliche Zusätze zugefügt werden können. Um eine leichte Löslichkeit des Emulsionspolymerisats in organischen Lösungsmittel zu gewährleisten, ist es nach DE-PS 2 512 238 bevorzugt, ein durch Sprühtrocknung aus der wäßrigen Dispersion des Emulsionspolymerisats gewonnenes Überzugsmittelpulver einzusetzen. Zur Auflösung können die für diesen Zweck üblichen organischen Lösungsmittel, wie niedere Alkohole und Ketone oder deren Gemische, eingesetzt werden. Die organisch gelöste Überzugsmasse kann in an sich bekannter Weise nach allen gebräuchlichen Verfahren zum Überziehen von Arzneiformen eingesetzt werden. Das Kesseldragierverfahren und das Wirbelschichtverfahren sind die gebräuchlichsten unter den in Betracht kommenden Verfahren.

Soweit es bei den bekannten Überzugsverfahren möglich oder gebräuchlich ist, den Überzug aus einer Vielzahl von Schichten allmählich aufzubringen, kann dies auch mit den erfindungsgemäßen Überzugsmassen geschehen. Dabei können die erfindungsgemäßen Überzugsmassen den gesamten Überzug oder gegebenenfalls eine Grundschicht, Zwischenschicht oder Deckschicht bilden wobei weitere Schichten mit einem anderen Löslichkeitsverhalten angewendet werden können. Derartige Einzelschichten in mehrschichtigen Überzügen können aus dem reinen Emulsionspolymerisat ohne weitere Zusätze aufgebaut sein. Auf die zahlreichen Methoden, durch eine gezielte Abfolge von Schichten mit unterschiedlichen Löslichkeitseigenschaften eine vorbestimmte Lösungs- und Wirkstoff- freigabecharakteristik im Magen-Darm-Trakt zu erreichen, braucht an dieser Stelle nicht näher eingegangen zu werden. Man kann verschiedene Schichten auch nach unterschiedlichen Verfahren aufbringen. So kann z. B. eine Matrixtablette, deren Kern aus im Sinne der Erfindung überzogenen Einzelpartikeln aufgebaut ist, mit einer äußeren Umhüllung aus andersartigem Material, z. B. einer Zuckerdragiermasse, umkleidet sein, oder umgekehrt.

Der Vielzahl der möglichen Anwendungsverfahren entspricht die Vielzahl der Arten von zu überziehenden Arzneiformen. Man kann Wirkstoffkristalle oder -granulate, Pillen, Dragee- und Tablettenkerne oder gefüllte Arzneimittelkapseln im Sinne der Erfindung überziehen. Als alleiniger Überzug oder als magensaftlösliche Schicht eines mehrschichtigen Überzugs kann die im Sinne der Erfindung aufgebrachte Schicht eine Dicke zwischen 5 bis 20 µm haben. Die erfindungsgemäßen Überzugsmassen lassen sich auch als Bindemittel zum Granulieren und Agglomerieren von Arzneistoffen einsetzen.

## Beispiele 1-17

Herstellung der Bindemittel

### Beispiel 1

In einem Polymerisationsgefäß mit Rührwerk und Heiz- bzw. Kühlmantel werden 0,4 g Natriumlaurylsulfat in 580 g Wasser gelöst und auf 80 °C erhitzt. Dazu gibt man eine Lösung von 0,7 g des Natriumsalzes der 4,4-Azobis-(4-cyanovaleriansäure) in 25 g Wasser.

In diese Vorlage läßt man innerhalb von 4 Stunden eine Emulsion, bestehend aus :

180 g N,N-Dimethylamino-2,2-dimethylpropylmethacrylat
210 g Methylmethacrylat
210 g Äthylacrylat
3 g Natriumlaurylsulfat
2 g Natriumsalz der 4,4-Azobis-(4-cyanovaleriansäure)
800 g Wasser

eintropfen. Die Temperatur im Reaktionsgefäß wird dabei auf 80 °C gehalten. Nach Abschluß des Zulaufs wird die Temperatur noch 2 weitere Stunden bei 80 °C belassen. Nach Abkühlen auf Raumtemperatur erhält man eine niedrigviskose, koagulatfreie Dispersion.

Festkörpergehalt : 30 Gew.-%
pH-Wert : 9,0

Minimale Filmbildungstemperatur : 24 °C
Weißpunkt : 18 °C

Beispiel 2

Man verfährt wie im Beispiel 1, konfektioniert die Dispersion jedoch nach Polymerisationsende und Abkühlen auf 30 °C durch Zutropfen einer Lösung von 14 g eines Additionsprodukts aus Isononylphenol und 100 Mol Äthylenoxid in 33 g Wasser.

Man erhält eine niedrigviskose Dispersion, Feststoffgehalt : 30 Gew.-% ; pH-Wert : 8,9 ; minimale Filmbildungstemperatur : 24 °C ; Weißpunkt : 18 °C.

Beispiel 3

Man konfektioniert wie in Beispiel 2 beschrieben, verwendet jedoch eine Lösung von 21 g des gleichen Anlagerungsprodukts in 50 g Wasser.

Es resultiert eine niedrigviskose Dispersion, Feststoffgehalt : 30 Gew.-% ; pH : 8,8 ; minimale Filmbildungstemperatur : 24 °C ; Weißpunkt : 18 °C.

Beispiel 4

Bei der in Beispiel 1 beschriebenen Arbeitsweise werden eingesetzt :

Vorlage

4 g Polyoxyäthylen-sorbitan-monooleat
0,7 g Natriumsalz der 4,4-Azobis-(4-cyanovaleriansäure)
600 g vollentsalztes Wasser

Emulsion

300 g N,N-Dimethylamino-2,2-dimethylpropylmethacrylat
180 g Äthylacrylat
120 g Methylmethacrylat
12 g Polyoxyäthylen-sorbitan-monooleat
2 g Natriumsalz der 4,4-Azobis-(4-cyanovaleriansäure)
800 g vollentsalztes Wasser

Es resultiert eine koagulatfreie, niedrigviskose Dispersion ; Feststoffgehalt : 29 Gew.-% ; minimale Filmbildungstemperatur : 15 °C ; Weißpunkt : 5 °C.

Beispiel 5

Man verfährt wie in Beispiel 1 unter Verwendung der dort angegebenen Vorlage. Die zugegebene Emulsion besteht aus :

180 g N,N-Dimethylamino-2,2-dimethylpropylmethacrylat
6 g Methacrylsäure (Gewichtsangabe als freie Säure, eingesetzt in Form des Natriumsalzes)
210 g Äthylacrylat
204 g Methylmethacrylat
3 g Natriumlaurylsulfat
2 g Natriumsalz der 4,4-Azobis-(4-cyanovaleriansäure)

Es resultiert eine koagulatfreie, niedrigviskose Dispersion ; Feststoffgehalt : 30 Gew.-% ; pH-Wert : 9,1 ; minimale Filmbildungstemperatur : 25 °C ; Weißpunkt : 18 °C.

Beispiel 6

Man verfährt wie in Beispiel 6 und konfektioniert die Dispersion nach Polymerisationsende bei 30 °C durch Zutropfen einer Lösung von 30 g eines Anlagerungsproduktes von 100 Mol Äthylenoxid an Isononylphenol, gelöst in 70 g Wasser.

Dispersionseigenschaften

Feststoffgehalt : 30 Gew.-%
pH-Wert : 8,7
Viskosität : 8 mPa · sec.

Beispiel 7

100 Gew.-Teile der Dispersion gemäß Beispiel 3 und 100 Gew.-Teile einer 30 %igen wäßrigen Dispersion eines Mischpolymerisats aus Äthylacrylat und Methylmethacrylat werden gemischt zu einer 30 %igen stabilen Dispersionsmischung ; pH-Wert : 8,5 ; minimale Filmbildungstemperatur ; 12 °C ; Weißpunkt : 0 °C.

Beispiel 8

Eine Dispersionsabmischung wird aus 100 Gew.-Teilen der Dispersion gemäß Beispiel 3 und 200 Gew.-Teilen einer 30 %igen wäßrigen Dispersion eines Alkylacrylat-Methylmethacrylat-Mischpolymerisats hergestellt. 30 %ige stabile Dispersionsmischung, pH-Wert : 8,3 ; minimale Filmbildungstemperatur : 9 °C ; Weißpunkt : 0 °C.

Beispiel 9

In einem Polymerisationsgefäß (wie in Beispiel 1) werden vorgelegt :

1,8 g Natriumlaurylsulfat
1,8 g Natriumsalz der 4,4-Azobis-(4-cyanovaleriansäure)
1 500 g Wasser

Dazu dosiert man innerhalb von 4 Stunden bei 80 °C eine Emulsion bestehend aus :

1 600 g N,N-Dimethylamino-2,2-dimethylpropylmethacrylat
720 g Methylmethacrylat
24 g 2-Äthylhexylthioglykolat.
16 g Natriumlaurylsulfat
5 g Natriumsalz der 4,4-Azobis-(4-cyanovaleriansäure)
2 350 g Wasser

Nach zweistündigem Nacherhitzen bei 80 °C wird auf Raumtemperatur gekühlt. Es resultiert eine gut filtrierbare etwa 40 %ige Dispersion mit niedriger Viskosität. Nach Entfernung geringer Mengen Koagulat (ca. 10 g) durch Filtration wird die Dispersion sprühgetrocknet. Man erhält ein Pulver aus weißen, nicht verglasten Körnchen mit etwa 30 μm Korndurchmesser. Schüttgewicht : 410 g/l ; Restfeuchte : 0,1 %.

Beispiele 10-16

Nach der Arbeitsweise gemäß den Beispielen 1 und 4 werden weitere Dispersionen mit geänderter Zusammensetzung der Monomerengemische hergestellt. Die Monomerengemische und Dispersionseigenschaften sind in Tabelle 1 angegeben. Die Dispersionen gemäß Beispielen 1 und 4 wurden zum Vergleich in die Tabelle eingefügt.

Tabelle 1

| Beisp. Nr. | Herstellungsverfahren nach | Monomerenzusammensetzung (Gew.-%) M | EA | MMA | MFT (°C) | WP (°C) |
|---|---|---|---|---|---|---|
| 10 | Beisp. 1 | 30 | 45 | 25 | 9 | 2 |
| 1 | — | 30 | 35 | 35 | 24 | 18 |
| 11 | Beisp. 1 | 30 | 25 | 45 | 43 | 35 |
| 12 | Beisp. 4 | 50 | 40 | 10 | 0 | 0 |
| 4 | — | 50 | 30 | 20 | 15 | 5 |
| 13 | Beisp. 4 | 50 | 25 | 25 | 26 | 19 |
| 14 | Beisp. 4 | 50 | 20 | 30 | 35 | 25 |
| 15 | Beisp. 1 | 90 | 10 | — | 25 | 16 |
| 16 | Beisp. 1 | 100 | — | — | 49 | 37 |

M = N,N-Dimethylamino-2,2-dimethylpropylmethacrylat
EA = Äthylacrylat
MMA = Methylmethacrylat
MFT = Minimale Filmbildungstemperatur
WP = Weißpunkt

Beispiele 17-19

Herstellung von Arzneimittelüberzügen

Beispiel 17

75 g der wäßrigen Dispersion gemäß Beispiel 1, enthaltend 22,5 g Lacktrockensubstanz, werden zusammen mit Farbpigmenten, Talkum, Weißpigment und Polywachs 6 000 zu einer 20,6 %igen Suspension vermischt und in einem Dragierkessel mit 35 cm Durchmesser auf 3 kg Tabletten aufgesprüht. Als Sprüheinrichtung wurde eine Luftdruckspritzpistole mit einem Düsendurchmesser von 1,0 mm verwendet. Die Sprühsuspension wurde mittels einer Schlauchpumpe (Schlauchdurchmesser : 3 mm) an die Sprühpistole befördert und mit einem Sprühdruck von 0,5 bar versprüht. Die Sprühgeschwindigkeit betrug während des kontinuierlichen Sprühprozesses 2,1 g Sprühsuspension pro min pro kg Tabletten. Während der Sprühauftrages wurde in die rotierenden Tabletten Warmluft von 70 °C (ca. 2 $m^3$/min) eingeblasen, so daß die Tablettentemperatur etwa 35 °C betrug.

Es resultierten Filmtabletten mit einem gleichmäßigen, glatten u. glänzenden Überzug, der in Wasser und künstlichem Magensaft BP in maximal 5 min zerfiel bzw. sich auflöste. Die Bruchfestigkeit erhöhte sich von 8 kp auf 10 kp. An Lacktrockensubstanz wurden 0,7 mg pro $cm^2$ Tablettenoberfläche bzw. 0,75 %, bezogen auf die Tablettenmenge aufgesprüht. An Gesamttrockensubstanz wurden 2,75 % der Tablettenmenge aufgetragen. Dies entspricht insgesamt einer Filmdicke von etwa 20 μm.

Die Dispersionen gemäß den Beispielen 2 und 3 wurden in der gleichen Weise verarbeitet und ergaben gleichwertige Überzüge.

Beispiel 18

75 g der Dispersion gemäß Beispiel 5, enthaltend 22,5 g Lacktrockensubstanz, wurden zusammen mit Farbpigmenten, Talkum, Weißpigment und Polywachs 6 000 zu einer Dragiersuspension verarbeitet und unter den gleichen Bedingungen wie in Beispiel 1 auf 3 kg Tabletten aufgesprüht. Die Sprühgeschwindigkeit betrug 2,3 g Sprühsuspension pro min und kg Tabletten.

Es resultierten glatte, gleichmäßige und glänzende Filmüberzüge, die in Wasser und künstlichem Magensaft innerhalb von 3 min zerfielen bzw. sich auflösten. Die Bruchfestigkeit der Tabletten erhöhte sich von 8 kp auf 9 kp.

An Lacktrockensubstanz sowie Gesamttrockensubstanz, wurden die gleichen Mengen wie in Beispiel 1 beschrieben aufgebracht.

Die Dispersionen gemäß den Beispielen 6 bis 8 wurden in gleicher Weise verarbeitet und ergaben gleichwertige Überzüge.

Beispiel 19

In einem Lösungsmittelgemisch aus 522 g Isopropanol und 348 g Aceton wurden 130 g des sprühgetrockneten Emulsionspolymerisats gemäß Beispiel 9 bei Raumtemperatur innerhalb von 25 min zu einer klaren, farblosen Lösung aufgelöst. 265 g dieser Lösung mit 33 g Lacktrockensubstanz wurde zusammen mit Talkum, Mg-Stearat, Weißpigment, Farbpigment und Polywachs 6 000 auf 3 kg Tabletten in einem Dragierkessel von 35 cm Durchmesser kontinuierlich aufgesprüht. Die Sprühsuspension enthielt 9,85 % Feststoffe. Als Sprüheinrichtung wurde eine Druckluftspritzpistole mit einem Düsendurchmesser von 1,0 mm verwendet. Die Sprühsuspension wurde mittels einer Schlauchpumpe (Schlauchinnendurchmesser : 3 mm) zur Sprühpistole befördert und anschließend mit 0,5 bar Druckluft auf die im Dragierkessel rotierenden Tabletten aufgesprüht. Während des Sprühprozesses wurde Warmluft von etwa 45 °C (2 $m^3$/min) in das Tablettenbett eingeblasen, so daß die Tablettentemperatur ca. 33 °C betrug. Es wurde mit einer Sprühgeschwindigkeit von 7 g Sprühsuspension pro min pro kg Tabletten gearbeitet.

Es resultierten glatte, gleichmäßige und glänzende Filmüberzüge, die in Wasser in maximal 5 min zerfielen und sich in künstlichem Magensaft BP innerhalb von 3 min auflösten. Die Bruchfestigkeit der Tabletten blieb unverändert.

An Lacktrockensubstanz wurden 1 mg pro $cm^2$ Tablettenoberfläche bzw. 1,1 %, bezogen auf die Tablettenmenge, aufgesprüht. An Gesamttrockensubstanz wurden 4,4 %, bezogen auf Tablettenmenge, aufgetragen. Dies entspricht insgesamt einer Schichtdicke von etwa 30 μm.

**Ansprüche**

1. In Magensaft lösliche oder quellbare Überzugsmasse für Arzneiformen, enthaltend als Bindemittel ein Emulsionspolymerisat aus Aminoalkylestern der Acryl- oder Methacrylsäure und gegebenenfalls aus weiteren Comonomeren, sowie weitere übliche flüssige oder feste Zusätze für Arzneimittelüberzüge,

dadurch gekennzeichnet, daß am Aufbau des Emulsionspolymerisats 5 bis 100 Gew.-% Einheiten beteiligt sind, die sich von Monomeren der Formel

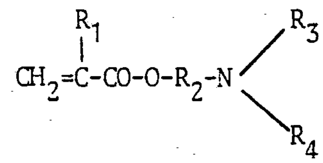

ableiten, wobei $R_1$ ein Wasserstoffatom oder eine niedere Alkylgruppe, $R_2$ eine Alkylengruppe mit wenigstens 3 in gerader Kette zwischen dem Amino-Stickstoffatom und dem Ester-Sauerstoffatom angeordneten Kohlenstoffatomen, von denen wenigstens eines tertiär oder quartär ist, und $R_3$ und $R_4$ niedere Alkylreste bedeuten oder zusammen mit dem Amino-Stickstoffatom einen heteroaliphatischen Ring bilden.

2. Überzugsmasse nach Anspruch 1, dadurch gekennzeichnet, daß sie eine wäßrige Phase mit einem pH-Wert über 7 enthält, worin das Emulsionspolymerisat in dispergierter Form vorliegt.

3. Überzugsmasse nach den Ansprüchen 1 und 2, dadurch gekennzeichnet, daß sie als weitere übliche Zusätze einen oder mehrere Stoffe aus der Gruppe : Pigmente, Füllstoffe, lösliche Farbstoffe, Weichmachungsmittel, Glanz- und Poliermittel, Geschmackszusätze, Lichtschutzzusätze, Entschäumungsmittel, Verlaufmittel, enthalten.

4. Verwendung des Emulsionspolymerisats gemäß Anspruch 1 als Bindemittel in Uberzugsmassen für Arzneiformen.

5. Verfahren zum Überziehen von Arzneiformen mit einer Überzugsmasse, enthaltend als Bindemittel ein Emulsionspolymerisat aus Aminoalkylestern der Acryl- oder Methacrylsäure und gegebenenfalls weiteren Comonomeren, dadurch gekennzeichnet, daß als Bindemittel ein Emulsionspolymerisat mit dem Kennzeichen gemäß Anspruch 1 eingesetzt wird.

**Claims**

1. Coating composition for pharmaceutical preparations which is soluble or swellable in the gastric juices, containing as binder an emulsion polymer obtained from aminoalkyl esters of acrylic or methacrylic acid and optionally other comonomers, and other conventional liquid or solid additives for coatings for pharmaceutical preparations, characterised in that from 5 to 100 % by weight of units derived from monomers of formula

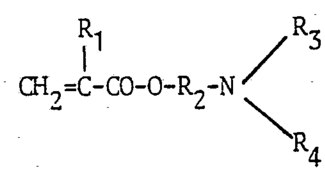

(wherein $R_1$ is a hydrogen atom or a lower alkyl group, $R_2$ is an alkylene group with at least 3 carbon atoms arranged in a straight chain between the amino nitrogen atom and the ester oxygen atom, at least one of these carbon atoms being tertiary or quaternary, and $R_3$ and $R_4$ represent lower alkyl groups or together with the amino nitrogen atom form a heteroaliphatic ring) participate in the synthesis of the emulsion polymer.

2. Coating composition as claimed in claim 1, characterised in that it contains an aqueous phase with a pH value of above 7, in which the emulsion polymer is present in dispersed form.

3. Coating composition as claimed in claims 1 and 2, characterised in that it contains, as other conventional additives, one or more substances from the group comprising pigments, fillers, soluble dyes, plasticisers, glosses and polishes, flavouring additives, light protection additives, antifoamers and flow agents.

4. Use of the emulsion polymer as claimed in claim 1 as a binder in coating compositions for pharmaceutical preparations.

5. Process for coating pharmaceutical preparations with a coating composition containing as binder an emulsion polymer obtained from aminoalkyl esters of acrylic or methacrylic acid and optionally other comonomers, characterised as in claim 1 is used as the binder.

**Revendications**

1. Matières de recouvrement pour médicaments, solubles ou gonflables dans le suc gastrique,

contenant, en tant que liant, un produit de polymérisation en émulsion d'esters aminoalcoyliques de l'acide acrylique ou méthacrylique et, le cas échéant, d'autres comonomères, ainsi que d'autres additifs liquides ou solides usuels pour revêtements de médicaments, caractérisées en ce qu'il entre, dans la constitution du produit de polymérisation en émulsion, 5 à 100 % en poids d'unités qui dérivent de monomères de formule

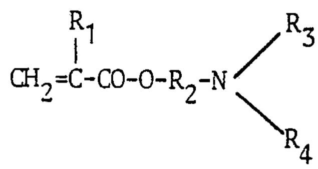

dans laquelle $R_1$ représente un atome d'hydrogène ou un groupe alcoyle inférieur, $R_2$ un groupe alcoylène comportant au moins 3 atomes de carbone disposés en chaîne droite entre l'atome d'azote du groupe amino et l'atome d'oxygène du groupe ester, atomes de carbone dont l'un au moins est tertiaire ou quaternaire, et $R_3$ et $R_4$ représentent des radicaux alcoyle inférieur ou forment, avec l'atome d'azote du groupe amino, un noyau hétéroaliphatique.

2. Matières de recouvrement selon la revendication 1, caractérisées en ce qu'elles contiennent une phase aqueuse ayant un pH supérieur à 7, dans laquelle le produit de polymérisation en émulsion est présent sous forme dispersée.

3. Matières de recouvrement selon la revendication 1 ou 2, caractérisées en ce qu'elles contiennent, en tant qu'autres additifs usuels, une ou plusieurs substances choisies dans les groupes suivants : pigments, charges, colorants solubles, plastifiants, agents de brillant et de poli, additifs aromatiques, additifs de protection contre la lumière, agents anti-mousse, produits nivelants.

4. Utilisation du produit de polymérisation en émulsion selon la revendication 1 comme liant dans des matières de recouvrement pour médicaments.

5. Procédé pour le revêtement de médicaments avec une matière de recouvrement qui contient, en tant que liant, un produit de polymérisation en émulsion formé d'esters aminoalcoyliques de l'acide acrylique ou méthacrylique et, le cas échéant, d'autres comonomères, caractérisé en ce qu'on utilise, en tant que liant, un produit de polymérisation en émulsion présentant les caractéristiques indiquées dans la revendication 1.